# EUROPEAN PATENT SPECIFICATION

(11) **EP 3 560 543 B1**
(45) Date of publication and mention of the grant of the patent: **18.05.2022**
(21) Application number: 18741676.3
(22) Date of filing: 18.01.2018
(51) Int. Cl.: A61N 1/24, A61M 31/00

(54) **DEVICE AND METHOD FOR ELECTROTHROMBOSIS**
VORRICHTUNG UND VERFAHREN FÜR ELEKTROTHROMBOSE
DISPOSITIF ET PROCÉDÉ POUR ÉLECTROTHROMBOSE

(30) Priority: 22.01.2017 CN 201710053933
(43) Date of publication of application: 30.10.2019
(73) Proprietor: Li, Youxiang, Beijing 100050 (CN); Jiang, Yuhua, Beijing 100050 (CN); Beijing Neurosurgical Institute, Beijing 100050 (CN)
(72) Inventor: LI, Youxiang, Beijing 100050 (CN); JIANG, Yuhua, Beijing 100050 (CN); ZHANG, Hongbin, Beijing 100050 (CN); GAO, Baofeng, Beijing 100050 (CN)
(74) Representative: Murgitroyd & Company
(86) International application number: PCT/CN2018/073182
(87) International publication number: WO 2018/133813

(56) References cited:
- EP-A1- 3 092 957
- EP-A2- 2 984 996
- WO-A1-82/00768
- WO-A2-02/098501
- CN-A- 105 363 117
- CN-A- 105 511 544
- CN-A- 106 100 371
- CN-A- 106 137 293
- CN-A- 106 137 294
- US-A- 5 643 254
- US-A- 5 855 578
- US-A1- 2013 079 693

## Description

### Technical Field

The application relates to an electrotherapeutic device for inducing thrombosis and a use of the device in therapy of intracranial aneurysms. The application relates to a use of a stent retriever (e.g. a Solitaire^{™} stent retriever) and a Traxcess^{™} series guide wire (e.g. a Traxcess^{™}-14 guide wire) in electrothrombosis, particularly in treatment of aneurysms. The application also relates to a power source specifically for electrothrombosis.

### Background Art

The concept of "electrothrombosis" appeared as early as two centuries ago. Its principle is to form blood clots by attracting negatively charged blood cells, platelets, coagulation factors, etc. in blood using positive charges present on a surface of electrically conductive materials. In 1824, Scudamore discovered that an anode electrode produced a thrombus, while a cathode electrode did not, which opened the prelude to the study of electrical thrombosis. In 1847, Ciniselli induced thrombosis by using a direct current needle to puncture aneurysms, making this technique be applied in the study of aneurysm therapy for the first time.

However, with the progress of medical technologies and the continuous improvement of human health needs, limitations of electrothrombosis have been increasingly emerging. Mullan et al. performed angiography 6 months after the operation on 12 cases of intracranial aneurysms treated with electrothrombosis in 1965, and the results showed that the thrombosis induced by electrothrombosis could not last long but only played a temporary role, failing to reach a lasting aneurysm embolization effect. In 1969, Mullan further pointed out upon research that a part of electric thrombus in the initial intracranial aneurysms could not successfully embolize the aneurysm within a reasonable time, and even was more likely to cause aneurysm hemorrhage because it changed stable blood circulation in the aneurysms. It thus can be seen that the mere electrothrombosis often leads to a failure of the electrothrombosis treatment for the intracranial aneurysms due to the instability of a fresh thrombus and an immediately coming fibrinolysis process.

By 2004, HenkesH et al. obtained the conclusion that the principle of electrothrombosis plays a uncertain role in the embolotherapy of Guglialmi detachable coil intracranial aneurysms by studying surface thrombosis with a Guglialmi detachable coil. Since then, the idea of realizing aneurysm embolization with electrothrombosis has been gradually given up in the industry and, in turn, conventional intravascular therapy represented by simple mechanical filling was adopted. After that, the rapid development of various embolization techniques and auxiliary embolization stents has further established the dominance of coil embolization on the intracranial aneurysms.

Although the conventional embolization methods, represented by the coil embolization, have a definite effect in embolizing aneurysms and inducing thrombosis, many problems are often encountered in clinical practice, including but not limited to:
(1) the effects of conventional intravascular treatment methods on clinical microaneurysms (especially those in which microcatheters cannot enter tumor cavities) are often not ideal;
(2) for blood blister-like aneurysms, the effects with the conventional intravascular embolotherapy are not ideal, the costs for a dense mesh stent and the like are too high, and the requirements on path vascular conditions are high;
(3) for large aneurysms, residual tumor necks are often encountered by conventional embolization, it is difficult to achieve dense embolization, even assisted by a stent, and a relapse often occurs quickly; the dense mesh stent is feasible, but expensive; and
(4) for smoky intra-group aneurysms and arteriovenous malformations (AVM) intra-group aneurysms, it is often difficult to keep peripheral small blood supply arteries in a conventional intravascular therapeutic process, resulting in insufficient blood supply for patients after operation. A device according to the preamble of claim 1 is known from US5643254.

To sum up, up to now, there is still a need for an effective, convenient and lasting thrombus induction method in the industry for aneurysms that are difficult to be treated with the traditional interventional therapy methods, so as to bring better therapy for patients with such aneurysms.

### Summary of the Invention

The invention is defined in claim 1.Any methods disclosed hereinafter do not form part of the scope of the invention. For aneurysms that are difficult to be treated with traditional interventional therapy methods, while other people in the industry are looking for other new therapeutic approaches, the inventors of the application unexpectedly started with and improved electrothrombosis which has already been abandoned, seeking out an effective method for electrothrombosis, and have made an unexpected combinations by using materials originally used for other purposes in the art, thus designing a device for the method above. The method and the device not only can well form a thrombus, but also are simple, convenient and easy to implement, can make the effect of thrombus lasting, and well solve the problems existing in the background art mentioned above.

In one aspect, the application relates to a method for thrombosis, including the following steps of:
1. a step of performing electrothrombosis, wherein constant-current direct current within a certain range is applied to attract negatively charged factors such as leukocytes, platelets, coagulation factors and the like in blood to induce thrombosis; and
2. a step of performing thrombus organization, wherein an electrothermal effect is further utilized to accelerate, denature and organized thrombosis so as to convert an unstable thrombus into a stable thrombus.

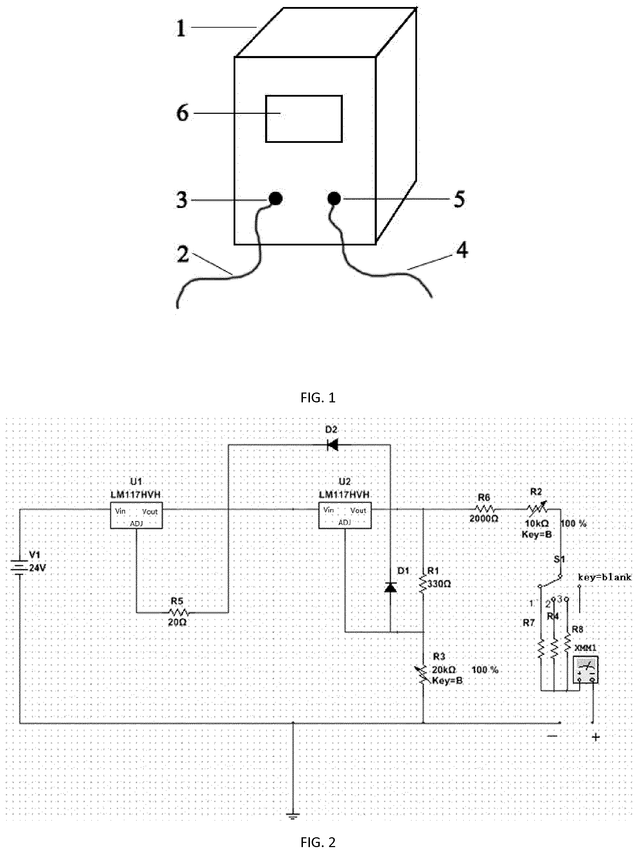

In another aspect, the application relates to a device for the implementation of the above-mentioned method. As shown in Fig. 1, a basic structure of the device includes (or consists of) a power source and a guide wire portion. The power source 1 provides constant current, the current is conducted from an anode 3 of the power source to aneurysms through the output guide wire 2 to induce thrombosis, and at the same time, the thrombus is denatured, organized and converted into the stable thrombus by utilizing the electrothermal effect. After that, the current flows into a cathode 5 of the power source through an input guide wire 4 to form a loop. The power source 1 is not limited in its own power supply mode, and power can be supplied either by direct current such as batteries or by connecting with an alternating current power source and then converting alternating current into direct current. The power source 1 is optionally provided with one or more panels 6 to display parameters such as current and voltage.

The method and the device of the application are convenient in material choosing, and simple and easy to implement. Since there is no stimulation from mechanical filling, the sizes of blood clots can be increased, thereby reducing factors causing rebleeding during and after an intravascular therapy operation on ruptured aneurysms. Particularly, the method and the device of the application limit a microcatheter or a micro guide wire within the aneurysm, thus reducing risks and difficulties brought by complicated operation caused by a tortuous vascular path and insufficient supporting force.

In addition, a thrombus is further induced to be denatured and organized on the basis of thrombosis, which prevents a process of fiber dissolution, and the thrombus is stabilized so as to reduce the probability of secondary rupture and hemorrhage of the ruptured aneurysms in the near future. At the same time, the method and the device of the application save a large amount of cost, and are especially important for patients in economically underdeveloped areas.

The application can either utilize a specialized power source and guide wire, or a Solitaire^{™} stent retriever and a Traxcess-14^{™} guide wire which are common in the market as the power source and the guide wire respectively. Although the original uses of the Solitaire^{™} stent retriever and the Traxcess-14^{™} guide wire have nothing to do with electrothrombosis, the applicant unexpectedly discovered that a combination of the two can achieve the purpose of electrothrombosis very well. The application in another aspect relates to a use of a stent retriever (e.g., a Solitaire^{™} stent retriever), a Traxcess^{™} series guide wire (e.g., a Traxcess-14^{™} guide wire), and a combination thereof in manufacturing a device for electric thrombosis.

In addition, in another embodiment, the applicant designs a constant current power source which is specially used as a power supply device for the electrothrombosis of the application. The power source is specifically designed based on the resistance of a human body and the current required by the electrothrombosis, and there is no such power source in the prior art that can output the current required by the electrothrombosis even after being connected with the human body like the power source designed by the application.

### Brief Description of the Drawings

Fig. 1 is a schematic diagram of a device of the application.
Fig. 2 is a circuit diagram of a power source for the application.
Fig. 3A is a radiographic image of Case 1 after being applied with an intracranial stent.
Fig. 3B is a radiographic image of Case 1 using a guide wire.
Fig. 3C is a radiographic image of Case 1 after being charged with electricity three times by a Solitaire^{™} stent retriever for 6 minutes in total.
Fig. 3D is a DSA image of Case 1 re-examined 6 months after electrotherapy.
Fig. 4A is a radiographic image of basilar artery perforating branch microaneurysms before electrothrombosis operation in Case 2.
Fig. 4B is a radiographic image of Case 2 after being charged with electricity three times by the Solitaire^{™} stent retriever for 6 minutes in total.
Fig. 35A is a radiographic image before electrothrombosis operation in Case 3.

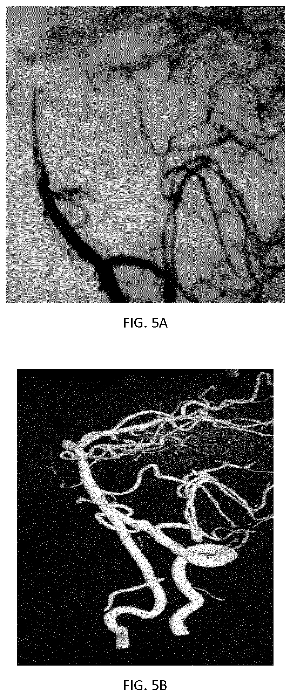

Fig. 5B is a radiographic image of Case 3 after being charged with electricity three times by the Solitaire^{™} stent retriever for 6 minutes in total.

### Detailed Description of the Invention

As mentioned above, a device of the application includes (or consists of) a power source and a guide wire portion.

The power source can be a commercially available constant current power source or constant voltage power source. The applicant discovered in clinical practice that the constant current power source has more advantages than the constant voltage power source as follows: although the constant voltage power source can provide constant output voltage, the stability and controllable output of current will be affected due to the individual differences in human body resistance of each person which cannot maintain stability at all times, while the constant current power source can directly provide constant current, which is more conducive to the stability of external conditions for thrombosis and the controllability of heat.

In a specific embodiment, the output current of the power source is about 0.1-50 mA, 0.2-20 mA, 0.5-10 mA or 0.8-5 mA. In another specific embodiment, the output current of the power source is about 0.8mA, 1mA, 1.5mA, 2mA, 3mA, or 5mA.

The applicant also surprisingly discovered that a commercially available stent retriever, such as a Solitaire^{™} stent retriever, can be used as a power source for the application. The stent retriever mainly uses the electrolysis principle in stent releasing, which has no relation with thrombosis in general medical practice. However, the inventors of the application unexpectedly discovered that the stent retriever can be advantageous in the electric thrombosis method of the application due to its stable and safe voltage (e.g., about 9 V), especially relatively constant current (e.g., about 0.8-1.0 mA) in the process of being power-supplied. Therefore, the application in another aspect relates to a use of a stent retriever (e.g., a Solitaire^{™} stent retriever) in electrothrombosis therapy and a use of the same in manufacturing a device for thrombosis.

In addition to using the stent retriever as a "guest" power source, other suitable power sources can also be used. For example, the inventors of the application design a power source which includes the following components: an internal power source, a voltage stabilizer, a diode, a first resistor, a second resistor, a third resistor, a first rheostat, an ammeter, an external electrode and a second rheostat, wherein the internal power source forms a power supply portion; the voltage stabilizer, the diode, the first resistor, the second resistor and the third resistor form a current control portion; the first rheostat and the second rheostat form a regulating portion; and the ammeter and the external electrode form an output portion. The regulating portion optionally further includes a position converter to switch the current between different switching positions, thereby constantly outputting corresponding current. Considering that subcutaneous resistance of a human body is generally not more than 500 Ω, the resistance ranges of the various resistors and rheostats can be determined as follows.

In the power supply portion, the internal power source can directly adopt a direct current power source or form a direct current power source by converting external alternating current into direct current. Direct current output voltage of the power source may be about 10 V, 2 V, 14 V, 16 V, 18 V, 20 V, 22 V, 24 V, 26 V, 28 V, 30 V, 32 V, 34 V, 36 V, 38 V or 40 V, for example, a 24 V direct current power source is used.

In the current control portion, the voltage stabilizer is configured to ensure that a circuit voltage of the output portion is relatively stable. The voltage stabilizer can adopt a common three-terminal voltage stabilizer in the market, for example, an LM117HVH^{™} three-terminal voltage stabilizer. An output terminal of the voltage stabilizer is divided into two branches which are respectively connected with the first resistor for voltage division and the third resistor for current limiting. The reason why the third resistor for current limiting needs to be provided is that the resistance values of different living bodies vary greatly, which may also lead to excessive current variation. Therefore, it is necessary to provide the resistor for current limiting here for safety. A resistance value of the first resistor may be about 200 Ω, 220 Ω,230 Ω,250 Ω,280 Ω,300 Ω,320 Ω,30 Ω,350 Ω,380 Ω or 400 Ω. A resistance value of the third resistor may be about 500 Ω,1000 Ω,1500 Ω,2000 Ω,2500 Ω,3000 Ω,3500 Ω or 4000 Ω.

In addition, the diode is connected to a third terminal of the voltage stabilizer and connected with the second resistor in series, thereby protecting the voltage stabilizer from being damaged due to excessively high output voltage. Specifically, when the output terminal of the voltage stabilizer is connected with a large capacitor and an input terminal thereof has a small voltage holding capacity, a situation that the potential of the output terminal is higher than that of the input terminal will occur after power off, therefore, the diode needs to be arranged to allow the capacitor at the output terminal of the voltage stabilizer to discharge to the input terminal so as to protect the voltage stabilizer. One or more (e.g., one, two or three) diodes may be connected in series according to the need for current regulation. A resistance value of the second resistor may be about 5 Ω,10 Ω,15 Ω,20 Ω,25 Ω,30 Ω,35 Ω,40 Ω.

In the regulating portion, the first rheostat is connected with the third resistor in series; the first rheostat and the third resistor together are connected with the second rheostat in parallel; and the first rheostat is configured to regulate current in a large range between different living bodies (for example, between different patients). The maximum resistance value of the first rheostat may be about 5 kΩ, 5.5 kΩ, 6 kΩ, 6.5 kΩ, 7 kΩ, 7. 5kΩ, 8 kΩ, 8.5 kΩ, 9 kΩ, 9.5 kΩ, 10 kΩ, 10.5 kΩ, 11 kΩ, 11.5 kΩ, 12 kΩ, 12.5 kΩ, 13 kΩ, 13.5 kΩ, 14 kΩ, 14.5 kΩ, or 15 kΩ. The first rheostat may be provided with several (for example, three) switching positions, and the resistance values of the switching positions may be about 0.5 kΩ, 1 kΩ, 1.5 kΩ, 2 kΩ, 2.5 kΩ, 3 kΩ, 3.5 kΩ, 4 kΩ, 4.5 kΩ, 5 kΩ, 5.5 kΩ, 6 kΩ, 6.5 kΩ, 7 kΩ, 7.5 kΩ, 8 kΩ, 8.5 kΩ, 9 kΩ, 9.5 kΩ, 10 kΩ, 10.5 kΩ, 11 kΩ, 11.5 kΩ, 12 kΩ, 12.5 kΩ, 13 kΩ, 13.5 kΩ, 14 kΩ, 14.5 kΩ, and 15 kΩ.

The second rheostat is configured to fine-tune the current when the living body fluctuates in a small range (for example, when operating on the same patient). The maximum resistance value of the second rheostat may be about 10 kΩ, 11 kΩ, 12 kΩ, 13 kΩ, 14 kΩ, 15 kΩ, 16 kΩ, 17 kΩ, 18 kΩ, 19 kΩ, 20 kΩ, 21 kΩ, 22 kΩ, 23 kΩ, 24 kΩ, 25 kΩ, 26 kΩ, 27 kΩ, 28 kΩ, 29 kΩ, or 30 kΩ. The second rheostat may also be provided with several (for example, three) switching positions, and the resistance values of the switching positions may be about 1 kQ, 2 kQ, 3 kΩ, 4 kΩ, 5 kΩ, 6 kΩ, 7 kΩ, 8 kΩ, 9 kΩ, 10 kΩ, 11 kΩ, 12 kΩ, 13 kΩ, 14 kΩ, 15 kΩ, 16 kΩ, 17 kΩ, 18 kΩ, 19 kΩ, 20 kΩ, 21 kΩ, 22 kΩ, 23 kΩ, 24 kΩ, 25 kΩ, 26 kΩ, 27 kΩ, 28 kΩ, 29 kΩ or 30 kΩ.

The regulating portion may also include the position converter connected with the first rheostat and the third resistor in series, and the function of current switching is realized by switching a control switch to different switching positions. The number of the switching positions may be two, three, four or five, etc. each of the switching positions is connected with a certain resistor so as to adjust the current within the range of the above-mentioned output current; and the resistance value of the resistor is related to the third resistor and the first rheostat, as well as to the resistance of different living bodies. For example, the resistance value of the resistor connected to each switching position can be about 0 kΩ, 0.3 kΩ, 0.5 kΩ, 1 kΩ, 2 kΩ, 3 kΩ, 4 kΩ, 5 kΩ, 6 kΩ, 7 kΩ, 8 kΩ, 9 kΩ, 10 kΩ, 11 kΩ, 12 kΩ, 13 kΩ, 14 kΩ, 15 kΩ, 16 kΩ, 17 kΩ, 18 kΩ, 19 kΩ, 20 kΩ, 21 kΩ, 22 kΩ, 23 kΩ, 24 kΩ, 25 kΩ, 26 kΩ, 27 kΩ, 28 kΩ, 29 kΩ or 30 kΩ respectively so as to control the output current at about 0.5 mA, 1 mA, 1.5 mA, 2 mA, 2.5 mA, 3 mA, 3.5 mA, 4 mA, 4.5 mA or 5 mA.

In the output portion, the ammeter is an ammeter commonly used in the industry, which is configured to indicate the current in electrothrombosis operation, and may act as a portion of the panels 6 described above. The measuring range of the ammeter matches the common current range of electrothrombosis, such as being 0-5 mA, 0-10 mA, 0-20 mA or 0-50 mA, etc. When in use, the first rheostat and/or the second rheostat can be regulated according to the readings of the ammeter. The external electrode corresponds to the above-mentioned anode 3 and cathode 5. The ammeter and the external electrode are connected with the first rheostat and the third resistor in series.

For the above-mention power source, Fig. 2 shows a specific embodiment. The internal power source is a 24 V direct current power source, an output terminal of which is connected with two LM117HVH^{™} three-terminal voltage stabilizers U1 and U2 which are connected in series; and an output terminal of U2 is divided into two branches which are respectively connected with a first resistor R1 (330Ω) and a third resistor R6 (2000Ω). In addition, one terminal of U2 is connected with two diodes D1 and D2 and a second resistor R5 (20Ω) in series to feed the current back to U1so as to protect U1 and U2. The first rheostat R2, the position converter, the ammeter XMM1 and the external electrode are connected with the third resistor R6 in series, and the series circuit is connected with the second rheostat R3 in parallel. The maximum resistance value of the first rheostat R2 is 10 kΩ; and the maximum value of the second rheostat R3 is 20 kΩ. The position converter has three switching positions which are connected with resistors R4, R7 and R8 respectively, so that the corresponding output currents are about 1 mA, 2 mA and 5 mA respectively.

In addition, the output guide wire of the application can adopt a guide wire commonly used in clinic. Preferably, the guide wire is provided with a head end with good conductivity, moderate heat generation and electrolytic resistance.

The inventors of the application unexpectedly discovered that commercially available Traxcess^{™} series guide wires, such as a Traxcess-14^{™} guide wire, are very suitable for use as an output guide wire of the application. The Traxcess^{™} guide wire was originally only used for general intravascular diagnosis or treatment by cooperating with a microcatheter, and there were no reports of its use in thrombosis. Due to its good conductivity and strong electrolytic resistance (e.g., the proximal of the Traxcess-14^{™} guide wire has an insulating coating over 140cm except for about 3cm at the tail end, which is beneficial to the concentration of positive charge to the head end covered by an inert platinum coil), the Traxcess^{™} guide wire is adapted to be used as the guide wire being charged with electricity in the method for electrothrombosis of the application. Therefore, the application in another aspect relates to a use of the Traxcess^{™} guide wire, especially the Traxcess-14^{™} guide wire, in electrothrombosis therapy and to a use in manufacturing a device for thrombosis.

The output guide wire of the application (for example, at the head end) is optionally equipped with a device for measuring a temperature or for temperature alarm, and/or an auxiliary device for introducing a microcatheter.

The input guide wire used in the application can be a guide wire commonly used in clinic, for example, a wire provided on an electrode of a common medical power source or a stent retriever can be used as the input guide wire. The input guide wire can be connected with a metal syringe needle and pricked under the skin of a human body (for example, under the skin of a thigh), or be tied at an appropriate position on the human body, or be pasted on the skin through a patch, thereby realizing circuit communication with the human body and forming a loop. Under the condition of not being pricked under the skin, one should pay attention to the fact that the resistance value of the human body will become larger. In order to ensure proper current, the parameters of the power source, such as voltage and internal resistance, should be regulated accordingly.

The application also relates to a use of a combination of a Solitaire^{™} stent retriever and a Traxcess-14^{™} guide wire in electrothrombosis therapy and a use in manufacturing a device for thrombosis.

### Examples

The following cases treated with the method and device of the application are aneurysms which are difficult to treat by conventional treatment methods, and the immediate postoperative effect and reexamination effect are very ideal.

### Materials and method

A commercially available Traxcess-14^{™} guide wire is used as an output guide wire, the tail end (namely, the conductive end without the coating) is connected to the anode of the Solitaire^{™} stent retriever, the head end of the guide wire is super-selected to an aneurysm cavity, and all guide wire portions inside of the body up to a tumor neck are isolated beyond the range of blood circulation by the microcatheter. In the thigh portion, a needle (a metal needle capable of conducting electricity, such as a common syringe needle) penetrates into the skin and is connected with a wire (serving as an input guide wire) on the cathode of the power source of the Solitaire^{™} stent retriever, so that positive charges converge at the aneurysm cavity, and current passes through a subcutaneous resistor of the human body and returns to the cathode of the release device via a thigh needle, thereby forming a complete loop (the principle is exactly the same as that of the release device).

During operative procedures, the head end of the guide wire is super-selected into the aneurysm cavity, the proximal end of the tumor neck is protected by the microcatheter in the whole process, the power source of the Solitaire^{™} stent retriever is powered on to realize the electrothrombosis operation in a intermittent way, and the current is controlled at about 1 mA when being powered on. Three times of such operation form one stage and one stage is followed by angiography until the effect is satisfactory.

### Case description

Case 1: a male who was 15 years old was injured after falling down when riding a motorcycle. He has multiple dissecting aneurysms and pseudoaneurysms at bilateral carotid arteries. With the above-mentioned operation, pseudoaneurysm to which the microcatheter cannot enter is subjected to eletrotherapy merely.

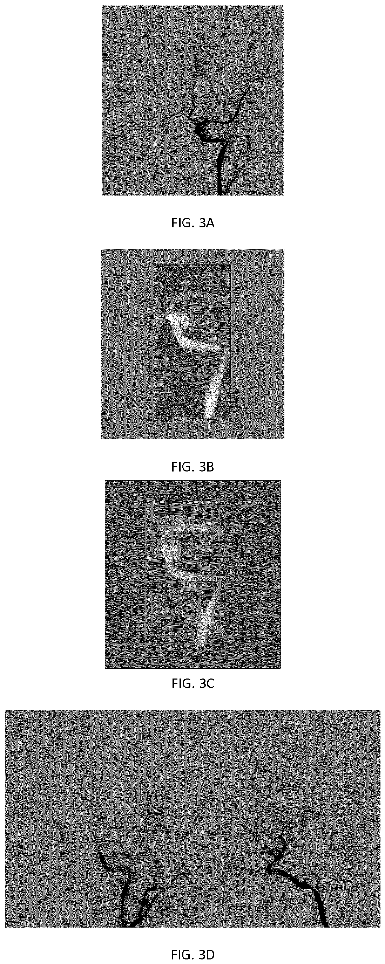

The therapeutic process is shown in Figs. 3A-3D. Fig. 3A shows that an ophthalmic artery segment pseudoaneurysm is still developed after being applied with an intracranial stent, and the conventional microcatheter cannot pass through a mesh. Fig. 3B shows that the Traxcess-14 guide wire is utilized, wherein the head end thereof can reach the tumor cavity and the microcatheter also can follow to the mesh of the intracranial stent. Fig. 3C shows that after the Solitaire^{™} stent retriever is powered on three times for 6 minutes in total, the tumor cavity is not developed obviously, indicating that an electric thrombus is well formed. Fig. 3D is a DSA image by reexamined 6 months after electric therapy, indicating that the electric thrombus is well maintained, and showing that the thrombus formed by the device and method of the application have a lasting effect.

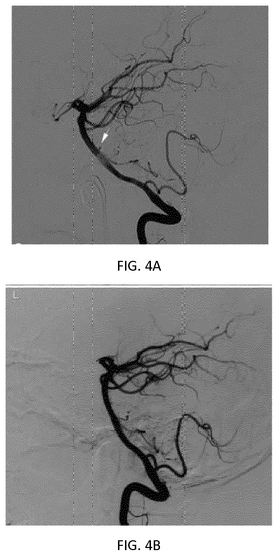

Case 2: a male who was 49 years old was admitted to hospital due to subarachnoid hemorrhage (SAH). As shown in the angiography in Fig. 4A, basilar artery perforating branch microaneurysms exist at the position indicated by the arrow. Since the pseudoaneurysm is so tiny that the conventional microcatheter cannot enter easily, the above-mentioned operation is adopted for electric therapy. After the Solitaire^{™} stent retriever is powered on three times for 6 minutes in total, the tumor cavity is not developed obviously. As shown in Fig. 4B, it shows that the electric thrombus is also well formed.

Case 3: a female who was 51 years old had a headache and had vomited for 10 days. Head CT showed hematocele in a prepontine cistern, an annular cistern and a fourth ventricle, and Head CTA showed the presence of extremely small aneurysms of the basilar truncus artiriosus. The angiography of the aneurysms is shown in Fig. 5A. After the Solitaire^{™} stent retriever is powered on 3 times for 6 minutes in total, the tumor cavity is not developed obviously. As shown in Fig. 5B, it indicates that the electric thrombus is also well formed.

The above-mentioned examples are only used to illustrate or explain the technical scheme of the application, and should not be construed as constituting any limitation to the application. Any improvement and modification of this application without creative labor shall also be deemed to fall within the scope of this application.

## Claims

1. An electrotherapeutic device for inducing thrombosis, comprising a power source (1), an output guide wire (2) and an input guide wire (4), wherein the output guide wire (2) is connected to an anode (3) of the power source (1); and the input guide wire (4) is connected to a cathode (5) of the power source (1) **characterized in that** the power source (1) comprises:
a power supply portion, comprising an internal power source, wherein the internal power source is a direct current power source, of which voltage is 10-40 V;
a current control portion, comprising a voltage stabilizer, a diode, a first resistor, a second resistor and a third resistor, wherein the voltage stabilizer is formed by connecting two three-terminal voltage stabilizers in series, and an input terminal of the voltage stabilizer is connected with the internal power source, and an output terminal thereof is divided into two branches which are respectively connected with the first resistor and the third resistor; the diode is connected with a third terminal of the voltage stabilizer and connected with the second resistor in series; a resistance value of the first resistor is 200-400 Ω; a resistance value of the second resistor is 5-40 Ω; and a resistance value of the third resistor is 500-4000 Ω;
a regulating portion, comprising a first rheostat and a second rheostat, wherein the first rheostat and the third resistor, which are connected in series, are connected with the second rheostat together in parallel; the maximum resistance value of the first rheostat is 5-15 kΩ; and the maximum resistance value of the second rheostat is 10-30 kΩ; and
an output portion, comprising an ammeter and an external electrode which are connected with the first rheostat and the third resistor in series.

2. The device of claim 1, further comprising a stent retriever connected to the output guide wire of the power source (1).

3. The device of claim 2, further comprising a Solitaire^{™} stent retriever connected to the output guide wire of the power source.

4. The device of claim 3, wherein the power source (1) further comprises a position converter, wherein the position converter is connected with the first rheostat in series and has two, three, four or five switching positions; and the resistance values of the resistors connected to the corresponding switching positions are 0-30 kΩ

5. The device of claim 3, wherein in the power source (1), the voltage of the internal power source is 24 V; the diode is formed by connecting two diodes in series; the resistance value of the first resistor is 330 Ω; the resistance value of the second resistor is 20 Ω; the resistance value of the third resistor is 2000 Ω; the maximum resistance value of the first rheostat is 10 kΩ; and the maximum resistance value of the second rheostat is 20 kΩ.

## Patentansprüche

1. Eine elektrotherapeutische Vorrichtung zum Einleiten von Thrombose, beinhaltend eine Stromquelle (1), einen Ausgangsführungsdraht (2) und einen Eingangsführungsdraht (4), wobei der Ausgangsführungsdraht (2) mit einer Anode (3) der Stromquelle (1) verbunden ist; und der Eingangsführungsdraht (4) mit einer Kathode (5) der Stromquelle (1) verbunden ist, **dadurch gekennzeichnet, dass** die Stromquelle (1) Folgendes beinhaltet:
einen Stromversorgungsabschnitt, der eine interne Stromquelle beinhaltet, wobei die interne Stromquelle eine Gleichstromquelle ist, deren Spannung 10-40 V beträgt;
einen Stromstärkesteuerungsabschnitt, der einen Spannungsstabilisator, eine Diode, einen ersten Widerstand, einen zweiten Widerstand und einen dritten Widerstand beinhaltet, wobei der Spannungsstabilisator durch Schalten von zwei dreipoligen Spannungsstabilisatoren in Reihe gebildet wird und ein Eingangsanschluss des Spannungsstabilisators mit der internen Stromquelle verbunden ist und ein Ausgangsanschluss davon in zwei Zweige geteilt ist, die jeweils mit dem ersten Widerstand und dem dritten Widerstand verbunden sind; wobei die Diode mit einem dritten Anschluss des Spannungsstabilisators verbunden ist und mit dem zweiten Widerstand in Reihe geschaltet ist; wobei ein Widerstandswert des ersten Widerstands 200-400 Ω beträgt; ein Widerstandswert des zweiten Widerstands 5-40 Ω beträgt; und
ein Widerstandswert des dritten Widerstands 500-4000 Ω beträgt;
einen Regulierungsabschnitt, der ein erstes Rheostat und ein zweites Rheostat beinhaltet, wobei das erste Rheostat und der dritte Widerstand, die in Reihe geschaltet sind, zusammen mit dem zweiten Rheostat parallel geschaltet sind; der maximale Widerstandswert des ersten Rheostats 5-15 kΩ beträgt; und der maximale Widerstandswert des zweiten Rheostats 10-30 kΩ beträgt; und
einen Ausgangsabschnitt, der ein Amperemeter und eine externe Elektrode beinhaltet, die mit dem ersten Rheostat und dem dritten Widerstand in Reihe geschaltet sind.

2. Vorrichtung gemäß Anspruch 1, ferner beinhaltend einen Stent-Retriever, der mit dem Ausgangsführungsdraht der Stromquelle (1) verbunden ist.

3. Vorrichtung gemäß Anspruch 2, ferner beinhaltend einen Stent-Retriever vom Typ Solitaire^{™}, der mit dem Ausgangsführungsdraht der Stromquelle verbunden ist.

4. Vorrichtung gemäß Anspruch 3, wobei die Stromquelle (1) ferner einen Stellungswandler beinhaltet, wobei der Stellungswandler mit dem ersten Rheostat in Reihe geschaltet ist und zwei, drei, vier oder fünf Schaltstellungen aufweist; und die Widerstandswerte der Widerstände, die mit den entsprechenden Schaltstellungen verbunden sind, 0-30 kΩ betragen.

5. Vorrichtung gemäß Anspruch 3, wobei in der Stromquelle (1) die Spannung der internen Stromquelle 24 V beträgt; die Diode durch Schalten von zwei Dioden in Reihe gebildet wird; der Widerstandswert des ersten Widerstands 330 Ω beträgt; der Widerstandswert des zweiten Widerstands 20 Ω beträgt; der Widerstandswert des dritten Widerstands 2000 Ω beträgt; der maximale Widerstandswert des ersten Rheostats 10 kΩ beträgt; und der maximale Widerstandswert des zweiten Rheostats 20 kΩ beträgt.

## Revendications

1. Un dispositif électrothérapeutique pour provoquer une thrombose, comprenant une source d'alimentation (1), un fil guide de sortie (2) et un fil guide d'entrée (4), le fil guide de sortie (2) étant connecté à une anode (3) de la source d'alimentation (1) ; et le fil guide d'entrée (4) étant connecté à une cathode (5) de la source d'alimentation (1), **caractérisé en ce que** la source d'alimentation (1) comprend :
une portion de bloc d'alimentation, comprenant une source d'alimentation interne, la source d'alimentation interne étant une source d'alimentation de courant continu, dont la tension est de 10 à 40 V ;
une portion de commande de courant, comprenant un stabilisateur de tension, une diode, une première résistance, une deuxième résistance et une troisième résistance, le stabilisateur de tension étant formé en connectant deux stabilisateurs de tension à trois bornes en série, et une borne d'entrée du stabilisateur de tension étant connectée à la source d'alimentation interne, et une borne de sortie de celui-ci étant divisée en deux branches qui sont respectivement connectées à la première résistance et à la troisième résistance ; la diode étant connectée à une troisième borne du stabilisateur de tension et connectée à la deuxième résistance en série ; une valeur de résistance de la première résistance étant de 200 à 400 Ω ; une valeur de résistance de la deuxième résistance étant de 5 à 40 Ω ; et une valeur de résistance de la troisième résistance étant de 500 à 4 000 Ω ;
une portion de régularisation, comprenant un premier rhéostat et un deuxième rhéostat, le premier rhéostat et la troisième résistance, lesquels sont connectés en série, étant connectés au deuxième rhéostat ensemble en parallèle ; la valeur de résistance maximale du premier rhéostat étant de 5 à 15 kΩ ; et la valeur de résistance maximale du deuxième rhéostat étant de 10 à 30 kΩ ; et
une portion de sortie, comprenant un ampèremètre et une électrode externe qui sont connectés au premier rhéostat et à la troisième résistance en série.

2. Le dispositif de la revendication 1, comprenant en outre un stent retriever connecté au fil guide de sortie de la source d'alimentation (1).

3. Le dispositif de la revendication 2, comprenant en outre un stent retriever Solitaire^{™} connecté au fil guide de sortie de la source d'alimentation.

4. Le dispositif de la revendication 3, dans lequel la source d'alimentation (1) comprend en outre un convertisseur de position, le convertisseur de position étant connecté au premier rhéostat en série et ayant deux, trois, quatre ou cinq positions de commutation ; et les valeurs de résistance des résistances connectées aux positions de commutation correspondantes étant de 0 à 30 kΩ.

5. Le dispositif de la revendication 3, dans lequel dans la source d'alimentation (1), la tension de la source d'alimentation interne est de 24 V ; la diode est formée en connectant deux diodes en série ; la valeur de résistance de la première résistance est de 330 Ω ; la valeur de résistance de la deuxième résistance est de 20 Ω ; la valeur de résistance de la troisième résistance est de 2 000 Ω ; la valeur de résistance maximale du premier rhéostat est de 10 kΩ ; et la valeur de résistance maximale du deuxième rhéostat est de 20 kΩ.
